# FASCICULE DE BREVET EUROPEEN

(11) **EP 2 895 110 B1**
(45) Date de publication et mention de la délivrance du brevet: **07.12.2022**
(21) Numéro de dépôt: 13759766.2
(22) Date de dépôt: 11.09.2013
(51) Int. Cl.: A61F 2/07, A61F 2/06

(54) **NÉCESSAIRE DE TRAITEMENT, DISPOSITIF DE TRAITEMENT ET PROCÉDÉ DE FABRICATION ASSOCIÉ**
BEHANDLUNGSKIT, BEHANDLUNGSVORRICHTUNG UND ZUGEHÖRIGES HERSTELLUNGSVERFAHREN
TREATMENT KIT, TREATMENT DEVICE, AND ASSOCIATED METHOD OF PRODUCTION

(30) Priorité: 11.09.2012 FR 1258524
(43) Date de publication de la demande: 22.07.2015
(73) Titulaire: Université de Strasbourg, 67000 Strasbourg (FR); Université de Haute Alsace, 68093 Mulhouse Cedex (FR); Hôpitaux Universitaires de Strasbourg (HUS), 67000 Strasbourg (FR)
(72) Inventeur: CHAKFÉ, Nabil, 67150 Hindisheim (FR); DURAND, Bernard, 68120 Pfastatt (FR); MARCHAND, Coralie, 68200 Mulhouse (FR)
(74) Mandataire: Merckling, Norbert
(86) Numéro de dépôt international: PCT/EP2013/068834
(87) Numéro de publication internationale: WO 2014/041028

(56) Documents cités:
- EP-A1- 1 836 995
- EP-A1- 1 847 237
- WO-A1-2005/034808
- WO-A1-2005/034810
- WO-A1-2011/051812
- US-A1- 2006 155 359
- US-B1- 6 585 762

## Description

La présente invention concerne un nécessaire de traitement comprenant :
- un premier implant comportant un premier corps tubulaire définissant une fenêtre;
- un deuxième implant comportant un deuxième corps tubulaire destiné à être disposé dans la fenêtre du premier corps tubulaire pour faire saillie par rapport au premier implant, le deuxième implant comportant un organe de retenue du deuxième implant par rapport au premier implant ;
- un anneau de retenue du deuxième implant rapporté sur le premier corps tubulaire dans la fenêtre et délimitant un passage d'introduction du deuxième implant.

Un tel nécessaire est destiné par exemple à être implanté dans une cavité d'un être vivant comportant un premier conduit, et au moins une branche faisant saillie transversalement à partir du premier conduit. La cavité est par exemple un conduit de circulation d'un fluide corporel, tel qu'un conduit de circulation du sang.

Dans un exemple, le conduit de circulation du sang est l'aorte, dans laquelle est implanté un premier implant. Un deuxième implant est déployé dans une artère branchée sur l'aorte, telle que l'artère iliaque, l'artère rénale, l'artère mésentérique supérieure, le tronc coeliaque et les troncs artériels supra-aortiques, etc...

En variante, le premier implant est destiné à être disposé dans la crosse aortique. Le deuxième implant est alors placé dans une des branches débouchant dans la crosse aortique, telle que l'artère carotide primitive gauche, l'artère sous-clavière gauche ou le tronc artériel brachiocéphalique.

D'autres applications sont possibles dans le système veineux, notamment dans la veine cave inférieure au niveau de la bifurcation iliaque, au niveau de l'abouchement des veines rénales et de la veine cave supérieure, au niveau de l'abouchement de ses branches collatérales.

Un tel nécessaire est généralement implanté dans un conduit de circulation du sang pour traiter des zones présentant des défauts ou des maladies, tels que des anévrismes ou les dissections.

Pour implanter le nécessaire, les techniques endoluminales sont actuellement préférées lorsqu'elles peuvent être mises en œuvre. En effet, ces techniques sont moins invasives pour le patient et réduisent les taux de mortalité et de morbidité, ainsi que la durée de séjour à l'hôpital.

Les techniques endoluminales fournissent d'excellents résultats pour le traitement des lésions de l'aorte thoracique descendante, qui est linéaire. Cependant, dans le cas de la crosse aortique, l'implantation du nécessaire par voie endoluminale présente un certain nombre de difficultés.

En premier lieu, le premier implant doit être suffisamment souple pour pouvoir s'adapter aux tortuosités de l'arbre artériel et se courber pour être amené de manière satisfaisante dans la crosse aortique puis déployé en se conformant à l'anatomie de ce vaisseau

Ensuite, plusieurs implants secondaires doivent être montés transversalement à travers des fenêtres ménagées dans le premier implant. Ces implants secondaires ont pour rôle de fixer axialement le premier implant en évitant le risque de migration, et de couvrir les branches artérielles supra-aortiques afin d'assurer une revascularisation de ces branches.

Pour déployer un nécessaire tel que précité dans un conduit de circulation du sang présentant une branche, il est connu tout d'abord de larguer le premier implant dans le conduit de circulation du sang. Une fenêtre ménagée dans le premier implant est disposée en regard de l'origine de chaque branche dans laquelle doit être implanté un deuxième implant. Puis, le deuxième implant est introduit dans la fenêtre du premier implant à travers la branche.

Le deuxième implant est ensuite déployé et est fixé sur le premier implant par l'intermédiaire d'au moins un organe de retenue. L'organe de retenue est par exemple une collerette déployable radialement à une extrémité du deuxième implant.

Pour assurer un bon fonctionnement du nécessaire une fois implanté dans l'organisme, il est nécessaire de réaliser une étanchéité quasi-totale entre le contour intérieur de la fenêtre ménagée dans le premier implant et le contour extérieur du deuxième implant.

A cet effet, US 2006/0155359 décrit un nécessaire du type précité, dans lequel le deuxième implant comporte à son extrémité liée au premier implant, une double collerette délimitant un étranglement annulaire permettant de recevoir la paroi extérieure du premier implant. Dans ce brevet, l'étanchéité est réalisée par les collerettes sur les faces internes et externes d'endoprothèse principale par l'application de surfaces les unes contre les autres.

WO 2005/034810 décrit un dispositif de traitement de sang comprenant un premier corps tubulaire définissant une fenêtre et un anneau de retenue pour un deuxième implant dans la fenêtre. WO2005/034810 divulgue aussi un nécessaire de traitement comprenant un premier et un deuxième implant et un anneau de retenue du deuxième implant sur le corps tubulaire du premier implant. WO2005/034810 représente l'état de la technique le plus proche pour l'objet des revendications indépendantes.

EP 1 836 995 décrit une prothèse tubulaire.

WO 2005/034808 décrit un stent ayant des fenêtres.

EP 1 847 237 décrit une greffe de stent.

US 6 585 762 décrit une greffe artérioveineuse.

WO 2011/051812 décrit une endoprothèse. Un tel nécessaire améliore l'étanchéité entre le premier implant et le deuxième implant, notamment lorsque le deuxième implant est sensiblement perpendiculaire au premier implant.

Toutefois dans certaines conformations anatomiques, le deuxième implant est incliné par rapport à un axe local du premier implant au niveau de la fenêtre, et n'est donc pas perpendiculaire à cet axe. De plus, dans certains cas, le contour extérieur du deuxième implant n'est pas conjugué au contour intérieur de la fenêtre. L'étanchéité entre le premier implant et le deuxième implant peut ne pas être totalement réalisée, ce qui est susceptible de conduire à des complications pour le patient.

Un but de l'invention est donc d'obtenir un nécessaire de traitement comportant un premier implant, et un deuxième implant implanté dans une fenêtre du premier implant, dans lequel l'étanchéité entre le premier implant et le deuxième implant est assurée, quelque soit le positionnement ou la conformation du deuxième implant par rapport au premier implant.

A cet effet, l'invention a pour objet un nécessaire selon la revendication 1.

Le nécessaire selon l'invention peut comprendre l'une ou plusieurs des caractéristiques selon les revendications dépendantes.

Le nécessaire selon l'invention comporte la caractéristique selon laquelle l'anneau de retenue est déformable élastiquement dans la fenêtre pour permettre une augmentation réversible d'au moins 20 %, avantageusement d'au moins 30 % du contour extérieur du passage d'introduction.

Le nécessaire selon l'invention peut comprendre l'une ou plusieurs des caractéristiques définies plus haut.

L'invention a également pour objet un dispositif de traitement selon la revendication 11.

Le dispositif selon l'invention comporte la caractéristique selon laquelle l'anneau de retenue est déformable élastiquement dans la fenêtre pour permettre une augmentation réversible d'au moins 20 %, avantageusement d'au moins 30 % du contour extérieur du passage d'introduction.

Le dispositif selon l'invention peut comprendre l'une ou plusieurs des caractéristiques définies plus haut.

L'invention a également pour objet un procédé de fabrication d'un dispositif selon la revendication 12.

Le procédé selon l'invention peut comprendre l'une ou plusieurs des caractéristiques selon les revendications dépendantes de la revendication 12.

L'invention sera mieux comprise à la lecture de la description qui va suivre, donnée uniquement à titre d'exemple, et faite en se référant aux dessins annexés, sur lesquels :
- la Figure 1 est une vue en perspective de trois-quarts face d'un premier nécessaire selon l'invention, dans lequel le premier implant a été monté dans le deuxième implant ;
- la Figure 2 est une vue, prise en coupe suivant un plan transversal, du nécessaire de la Figure 1 ;
- la Figure 3 est une vue en perspective du premier implant de la Figure 1 ;
- la Figure 4 est une vue de dessus de l'implant de la Figure 3, l'anneau de retenue occupant une configuration de repos ;
- la Figure 5 est une vue analogue à la Figure 4, l'anneau de retenue occupant une configuration dilatée ;
- la Figure 6 est une vue en perspective partielle de l'anneau de retenue ;
- la Figure 7 est une vue de dessus du ressort disposé dans l'anneau de retenue ;
- la Figure 8 est une vue schématique d'un dispositif destiné à fabriquer l'anneau de la Figure 6 ;
- la Figure 9 est une vue analogue à la Figure 8 lors de la fabrication de l'anneau ;
- la Figure 10 est une vue schématique en coupe partielle d'un deuxième nécessaire selon l'invention, en cours d'implantation dans un conduit coudé de circulation du sang ; dans un exemple possible de cathétérisation par l'intérieur de l'endoprothèse principale.
- la Figure 11 est une vue schématique en coupe partielle du deuxième nécessaire selon l'invention, implanté dans un anévrisme de l'aorte thoracique descendante nécessitant de couvrir l'artère sous-clavière gauche et/ou l'artère carotide primitive gauche ;
- la Figure 12 est une vue schématique en coupe partielle du deuxième nécessaire selon l'invention, implanté dans un anévrisme de la crosse aortique où les ostia des troncs supra-aortiques sont distants des fenêtres ;
- la Figure 13 est une vue, prise en coupe partielle, d'un troisième nécessaire selon l'invention, une fois implanté dans un conduit de circulation du sang en forme de Y ;
- la Figure 14 illustre le troisième nécessaire selon l'invention, implanté dans un anévrisme illiaque unilatéral sans collet proximal.
- la Figure 15 est une vue, prise en coupe partielle d'un quatrième nécessaire selon l'invention ;
- la Figure 16 illustre une variante du quatrième nécessaire selon l'invention ;
- la Figure 17 illustre un détail de la variante de la Figure 16 ;
- la Figure 18 illustre une variante de deuxième implant pouvant être disposé dans un premier implant selon l'invention ;
- la Figure 19 illustre en coupe partielle l'implant de la Figure 18 ;
- la Figure 20 illustre l'implant de la Figure 18 disposé dans le premier implant ;
- la Figure 21 illustre un exemple d'armature pour une autre variante du deuxième implant ;
- la Figure 22 est une vue analogue à la Figure 20 de l'autre variante du deuxième implant.

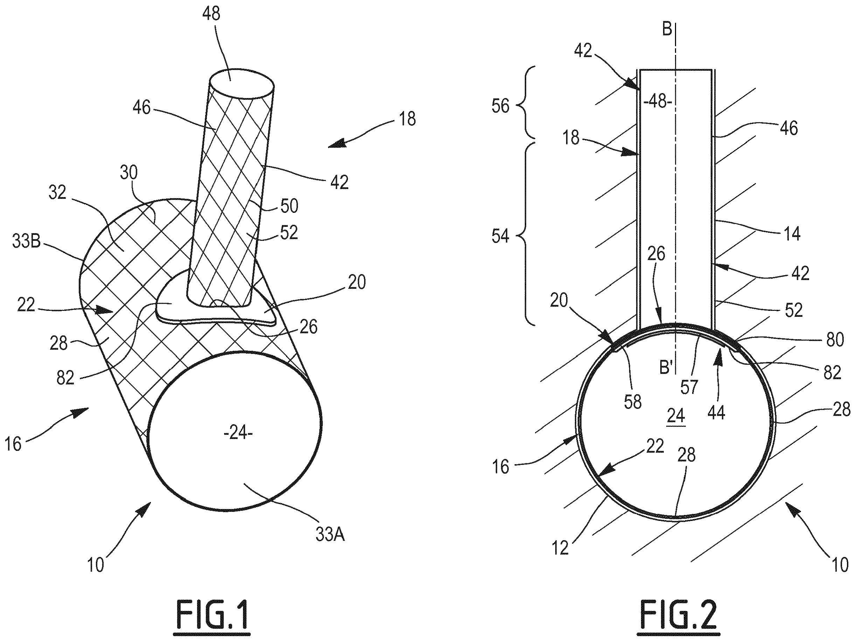

Un premier nécessaire 10 selon l'invention est illustré par les Figures 1 à 7. Le premier nécessaire 10 est destiné à être implanté dans une cavité intérieure définie dans le corps d'un être humain ou animal.

En référence à la Figure 2, la cavité comprend un tronçon principal 12 tubulaire et une branche 14 débouchant dans le tronçon principal 12.

Le tronçon principal 12 et la branche 14 sont par exemple des conduits de circulation du sang dans le système vasculaire d'un être humain ou animal. Le tronçon principal 12 est par exemple une artère ou une veine, et la branche 14 est un conduit de circulation du sang débouchant dans l'artère ou dans la veine.

En particulier, le tronçon 12 peut être l'aorte, notamment au niveau de la crosse aortique, ou un tronçon linéaire de l'aorte dans lequel sont connectés des segments d'endoprothèses abdominales.

Comme illustré par les Figures 1 et 2, le nécessaire 10 comporte un premier implant 16 destiné à être implanté dans le tronçon principal 12, et un deuxième implant 18. Dans cet exemple, le deuxième implant 18 est destiné à être assemblé transversalement sur le premier implant 16 pour faire saillie à partir du premier implant 16 dans la branche 14.

Selon l'invention, le nécessaire 10 comporte en outre un anneau 20 élastique, monté sur le premier implant 16 pour réaliser une étanchéité autour du deuxième implant 18.

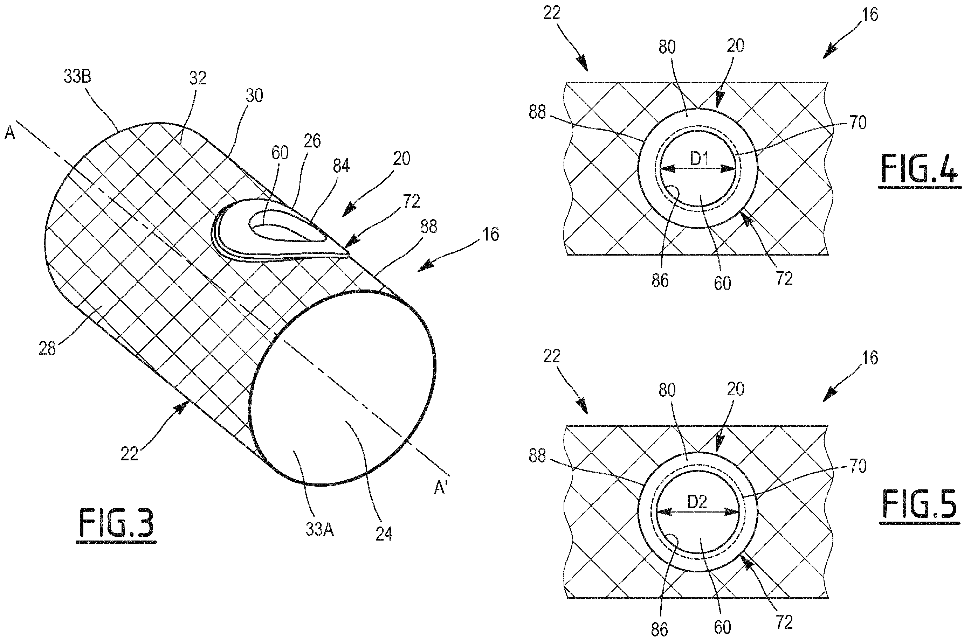

En référence à la Figure 3, le premier implant 16 comporte un premier corps tubulaire 22 définissant un passage central 24 de circulation d'un fluide corporel et une fenêtre latérale 26 d'insertion du deuxième implant 18 s'ouvrant dans le passage central 24.

Le corps tubulaire 22 présente une forme tubulaire d'axe central A-A'. Il comporte une paroi tubulaire 28 formée avantageusement d'une armature ajourée 30 recouverte d'une couche 32 de revêtement.

L'armature 30 est par exemple formée à partir d'au moins un fil élastique, réalisé par exemple en métal à mémoire de forme, tel qu'en Nitinol, ou en polymère.

Le ou chaque fil est par exemple conformé en ziz-zag ou sous forme d'un treillis définissant des mailles.

La couche de revêtement 32 obture des ouvertures intermédiaires définies entre les tronçons de fil. Elle délimite de manière étanche le passage central 24 sur toute la longueur du corps 22, à l'exception de la fenêtre 26.

La couche de revêtement 32 est par exemple formée à base d'un film étanche aux liquides, présentant par exemple une épaisseur inférieure à 1 mm.

Le film est par exemple réalisé en un polymère silicone, ou en un polymère fluoré tel que du PTFE.

Le passage central 24 est délimité de manière étanche par la paroi périphérique 28. Il débouche axialement de part et d'autre du corps tubulaire 22 par une ouverture proximale 33A et par une ouverture distale 33B. Il débouche transversalement à travers la fenêtre 26.

La fenêtre 26 est ménagée transversalement dans la paroi périphérique à l'écart des ouvertures 33A, 33B.

Le premier implant 16 est déployable entre une configuration contractée d'amenée vers son lieu d'implantation (non représentée) et une configuration expansée d'implantation.

Dans la configuration contractée, l'implant 16 est contracté radialement vers l'axe A-A' et présente une étendue radiale minimale, en vue de son introduction dans le tronçon principal 12.

Dans la configuration expansée, il présente un diamètre maximal.

Dans un mode de réalisation avantageux, le premier implant 16 est autoexpansible. Il est alors déployable spontanément depuis sa configuration contractée vers sa configuration expansée, sa configuration expansée constituant sa configuration de repos.

Comme illustré par les Figures 1 et 2, le deuxième implant 18 comprend un deuxième corps tubulaire 42 et au moins un organe radial 44 de retenue du deuxième implant 18 dans le premier implant 16.

Le deuxième corps tubulaire 42 comporte une paroi tubulaire 46 définissant un passage central auxiliaire 48.

Comme pour la paroi tubulaire 28 du premier corps 22, la paroi tubulaire 46 du deuxième corps 42 comporte une armature ajourée 50 recouverte d'une couche de revêtement 52. L'armature 50 est par exemple formée par au moins un fil conformé en zig-zag, ou par un treillis de fils définissant des mailles.

Dans l'exemple représenté sur la Figure 2, la couche de revêtement 52 couvre une première région proximale 54 du corps tubulaire 42 située en contact avec l'organe de retenue 44 pour rendre cette première région 54 étanche au liquide.

Par contre, au moins une région distale 56 située à l'opposé de l'organe de retenue 44 est découverte pour autoriser le passage de liquide. Ceci assure une vascularisation de la branche 14 lors de l'implantation du deuxième implant 18.

Dans l'exemple représenté sur la Figure 2, l'organe radial de retenue 44 fait saillie radialement à partir d'un bord proximal du deuxième corps tubulaire 42. Dans cet exemple, l'organe radial 44 est formé par une collerette 58 faisant saillie radialement par rapport à un axe B-B' du deuxième corps tubulaire 42.

La collerette 58 est par exemple continue sur toute la périphérie du bord distal 57 autour de l'axe B-B'. En variante, la collerette 58 est formée par une pluralité de doigts disjoints discontinus.

L'organe radial 44 fait ainsi saillie radialement autour de l'axe B-B' par rapport au deuxième corps tubulaire 42, avantageusement perpendiculairement par rapport à l'axe B-B'.

Une fois le deuxième implant 18 monté sur le premier implant 16, l'organe radial 44 est appliqué contre une surface intérieure du premier corps tubulaire 22 située autour de la fenêtre 26 pour empêcher le déplacement radial vers l'extérieur du deuxième implant 18 par rapport au premier implant 16.

Le deuxième implant 18 présente ainsi une forme générale de T et est désigné par le terme « T-stent ».

Des exemples de deuxièmes implants 18 sont décrits dans la demande de brevet WO 2011/051812 de la Demanderesse.

Comme pour le premier implant 16, le deuxième implant 18 est déformable entre une configuration rétractée d'introduction et une configuration déployée implantée dans la branche 14 et dans la fenêtre 26 du premier implant, telle que représentée sur la Figure 2. Avantageusement, le deuxième implant 18 est autoexpansible.

Dans la configuration rétractée, le deuxième implant 18 présente une étendue radiale minimale. Dans cette configuration, l'organe radial 44 est contracté radialement au voisinage de l'axe B-B'. Le deuxième implant 18 est apte à être convoyé à travers le passage central 24 et la fenêtre 26.

Dans la configuration déployée, le deuxième corps tubulaire 42 présente une étendue radiale maximale autour de l'axe B-B'. L'organe radial 44 est déployé transversalement par rapport à l'axe B-B'.

Comme illustré par les Figures 3 à 7, l'anneau élastique 20 est rapporté sur la paroi tubulaire 28 du corps tubulaire 22 autour de la fenêtre 26. L'anneau 20 délimite un passage central 60 d'insertion du deuxième implant 18 dont l'étendue et le contour sont propres à se conformer à l'étendue et au contour extérieur du deuxième implant 18.

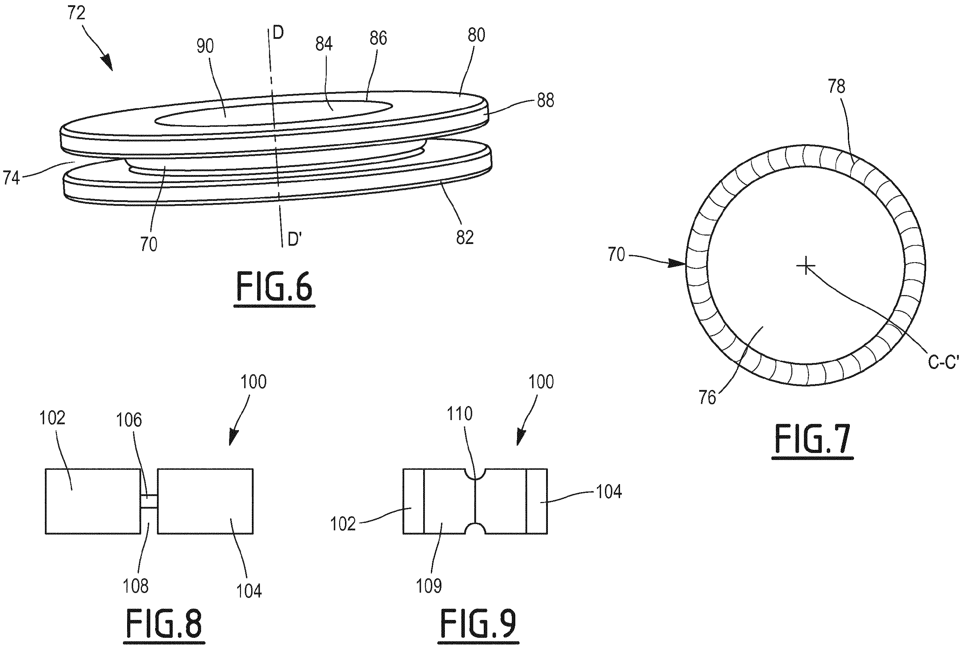

Dans l'exemple illustré sur les Figures 6 à 7, l'anneau élastique 20 comporte un organe élastique formé par un ressort 70 circonférentiel et une enveloppe 72 définissant un logement 74 de réception du ressort 70.

Le ressort 70 s'étend autour d'un axe central C-C'. Il présente par exemple une forme de tore. Avantageusement, le ressort 70 est fermé sur lui-même. Le ressort 70 définit une ouverture centrale 76.

Dans l'exemple représenté sur la Figure 7, le ressort 70 est un ressort hélicoïdal présentant une pluralité de spires 78 s'étendant sensiblement radialement par rapport à l'axe central C-C'. Il est formé à la base d'un brin filiforme métallique enroulé en hélice sur lui-même.

Les spires du ressort hélicoïdal 70 définissent une cavité torique intérieure.

Avantageusement, le rapport entre le diamètre des spires 78 et le diamètre du brin est compris entre 5 et 15, avantageusement entre 8 et 12, et notamment environ égal à 10.

Typiquement, le diamètre du brin est compris entre 0,1 mm et 0,2 mm, et le diamètre de chaque spire 78 est compris entre 0,6 mm et 2 mm.

De même, le rapport entre le diamètre de l'ouverture centrale 76 et le diamètre de chaque spire 78 est compris entre 8 et 15.

Le ressort 70 est déformable élastiquement, radialement par rapport à l'axe C-C'.

Il est déformable de manière réversible sur une plage de déformation élastique réversible supérieure à 20%, avantageusement d'au moins 30%. Ainsi, le périmètre du ressort 70 est apte à augmenter de manière élastique et réversible d'au moins 20%, avantageusement d'au moins 30%, ce qui augmente le diamètre de l'ouverture centrale 76.

Le module élastique du ressort 70, tel que mesuré par dynamométrie est par exemple compris entre 4 N/mm et 8 N/mm.

Comme illustré par la Figure 6, l'enveloppe 72 s'étend autour d'un axe central D-D'. Elle comporte une collerette extérieure 80, une collerette intérieure 82 et une paroi tubulaire 84 centrale raccordant les bords intérieurs 86 des collerettes 80, 82.

Les collerettes 80, 82 et la paroi centrale 84 sont avantageusement venues de matière. Elles sont par exemple formées en tissu tressé ou tricoté, tel qu'un tissu de poly éthylènetéréphtalate ou de polytétrafluoéthylène.

La collerette 82 est rapportée par exemple par son bord extérieur 88 sur une surface extérieure de la paroi 28 du corps tubulaire 22. La collerette intérieure 82 est rapportée par exemple par son bord extérieur 88 sur une surface intérieure de la paroi tubulaire 28 autour de la fenêtre 26. La paroi tubulaire 28 est ainsi reçue partiellement dans le logement 74 défini entre les collerettes 80, 82.

La paroi centrale 84 s'étend entre les collerettes 80, 82 à partir de leurs bords intérieurs 86.

Le logement 74 présente ainsi une forme de gorge annulaire s'étendant autour de l'axe D-D'. Le logement 74 est obturé radialement vers l'axe D-D' par la paroi centrale 84. Il est obturé extérieurement par la collerette 80 et intérieurement par la collerette 82.

Lorsque l'anneau 20 est rapporté sur la paroi tubulaire 28, le logement 74 est obturé radialement à l'écart de l'axe D-D' par le pourtour de la fenêtre 26.

Le ressort 70 est reçu dans le logement 74 entre la paroi centrale 84, et les collerettes 80, 82. Il est disposé avantageusement en appui contre la paroi centrale 84.

L'anneau 20 définit ainsi dans la fenêtre 26 le passage 60 d'introduction du deuxième implant 18, dont le contour extérieur et l'étendue sont adaptables au contour extérieur du deuxième implant 18. Le passage d'introduction 60 s'étend axialement à travers l'enveloppe 70, dans la paroi centrale 84. Le ressort 70 est monté autour du passage 60.

Ainsi, l'anneau 20 est déformable élastiquement dans la fenêtre 26 pour permettre une augmentation réversible d'au moins 20%, avantageusement d'au moins 30%, du pourtour extérieur du passage d'introduction 60.

Cette déformation réversible résulte notamment de la présence du ressort 70 qui peut s'étendre radialement de manière réversible par écartement de ses spires 78.

Ainsi, le deuxième implant 18 est mobile par rapport au premier implant 16 entre une position de repos, située à l'écart de la fenêtre latérale 26, et une position active implantée dans la fenêtre latérale 26.

Dans la position active implantée, l'anneau 20 est déformé élastiquement autour du deuxième corps tubulaire 42. Le contour du passage central 60 présente alors une forme conjuguée au contour extérieur du deuxième corps 42 au contact de l'anneau élastique 20.

Pour garantir une bonne étanchéité, le contour extérieur du deuxième corps 42 dans sa configuration déployée présente une étendue supérieure à l'étendue du contour intérieur du passage d'introduction 60 en l'absence de déformation du ressort 70.

Lorsque le deuxième implant 18 est reçu dans le passage d'introduction 60, une force radiale de retenue et d'étanchéité s'applique contre la surface extérieure du deuxième corps tubulaire 42, quelle que soit la conformation spatiale du deuxième corps tubulaire 42 par rapport au premier corps tubulaire 22.

En particulier, l'étanchéité est réalisée sur toute la périphérie du passage 60.

L'anneau 20 garantit donc une étanchéité engendrant une force significative et continue à l'interface entre le premier implant 16 et le deuxième implant 18 dans la fenêtre 26, l'anneau 20 formant une lèvre continue du premier implant 16 appliquée sur une zone de connexion du deuxième implant 18.

Par ailleurs, le deuxième implant 18 est retenu de manière extrêmement robuste dans le premier implant 16, puisque la force nécessaire à l'arrachement du deuxième implant 18 hors de l'anneau 20 est supérieure à 20N, et est notamment de 25N environ.

Un exemple de procédé de fabrication d'un anneau 20 rapporté sur le premier implant 16 va maintenant être décrit.

Ce procédé est par exemple mis en œuvre à l'aide d'un dispositif 100 représenté sur la Figure 8 et sur la Figure 9.

Le dispositif 100 comporte un premier mandrin 102 et un deuxième mandrin 104 raccordés entre eux par une tige intermédiaire 106. Les mandrins 102, 104 sont montés sur la tige 106 avec un écartement donné réglable.

Dans un première mode de réalisation, un tube 109 de matériau souple destiné à former l'enveloppe 72 est découpé. Le tube 109 est engagé autour du premier mandrin 102, de l'espace intermédiaire 108 entre les mandrins, dans lequel est logée la tige 106, et du deuxième mandrin 104.

Puis, un lien est noué autour du tube 109 au niveau de l'espace intermédiaire 108 pour former un étranglement 110 dans le tube 109.

Puis, le dispositif 100 est plongé dans un liquide pour figer la déformation du tube 109 causée par le lien. Ensuite le lien est à nouveau resserré, et la distance entre les deux mandrins 102, 104 est diminuée au minimum.

La conformation du tube 109 est alors figée, comme décrit précédemment. Le tube 109 déformé est ensuite séché, et le surplus de matériau est coupé à la périphérie pour former les collerettes 80, 82.

Le ressort 70 est alors engagé dans le logement 74 formé par l'étranglement 110, puis les collerettes 80, 82 sont fixées sur la paroi tubulaire 22 du premier implant 16.

Puis, un revêtement étanche est appliqué sur l'enveloppe 70 pour garantir son étanchéité au liquide. Le revêtement étanche est par exemple formé de silicone imprégnant la structure.

Dans une variante, le tube 109 est formé par une bande enroulée autour des mandrins 102, 104 en regard de l'espace intermédiaire 108.

L'utilisation du premier nécessaire 10 selon l'invention va maintenant être décrite. Initialement, le premier implant 16 est introduit dans le corps, par exemple par voie endoluminale.

A cet effet, le premier implant 16 est chargé dans sa configuration rétractée sur un premier dispositif de largage (non représenté) et est convoyé jusqu'au tronçon principal 12.

Une fois dans le tronçon principal 12, la fenêtre 26 est disposée en regard de la branche 14.

Le caractère radioopaque du ressort 70 est avantageusement utilisé pour positionner angulairement et axialement la fenêtre 26 par rapport à la branche 14.

Ceci étant fait, le premier corps tubulaire 22 est déployé jusqu'à sa configuration expansée, pour venir en appui contre la paroi du tronçon principal 12.

Puis, le deuxième implant 18, chargé sur un deuxième dispositif de largage (non représenté) est amené jusqu'au premier implant 16, par exemple par voie endoluminale.

Dans un premier mode de réalisation, le deuxième implant 18 est convoyé jusqu'au tronçon principal 12, puis est introduit dans le passage central 24 du premier corps tubulaire 22.

Le deuxième dispositif de largage est alors guidé sur un guide chirurgical pour extraire partiellement le deuxième implant 18 hors du premier implant 16 à travers la fenêtre 26. A cet effet, au moins une partie du deuxième corps tubulaire 42 est engagée à travers le passage central 60 de l'anneau élastique 20.

Le bord distal du deuxième implant 18 est maintenu dans le passage central 24 du premier implant 16.

Puis, le deuxième corps tubulaire 42 est passé dans sa configuration déployée, au moins au niveau de son extrémité proximale.

L'organe radial 44 se déploie alors radialement par rapport au corps tubulaire 42 et s'engage dans le passage central 26 en regard de l'anneau élastique 20.

L'anneau élastique 20 étant déformable élastiquement sur une grande plage de déformation, il adapte automatiquement son contour intérieur à celui du contour extérieur du deuxième implant 18, quelle que soit la configuration spatiale et l'orientation relative du deuxième implant 18 par rapport au premier implant 16.

L'étanchéité est donc réalisée de manière sûre par l'anneau élastique 20 rapporté sur le premier implant 16, ce qui garantit qu'aucune fuite de fluide corporel n'existe entre le premier implant 16 et le deuxième implant 18.

Puis, la partie distale du deuxième implant 18 est déployée dans la branche 14 et est appliquée contre la paroi de la branche 14.

Dans un deuxième mode d'implantation du deuxième implant 18, le deuxième implant 18 est introduit à travers la branche 14 vers le premier implant 16, pour introduire l'organe radial 44 en configuration contractée dans le passage central 24. Puis, l'organe radial 44 est déployé et est placé en butée contre une surface intérieure de la paroi périphérique 28 autour de la fenêtre 26.

Ensuite, le deuxième corps tubulaire 42 est déployé progressivement depuis l'organe radial 44 vers son extrémité distale.

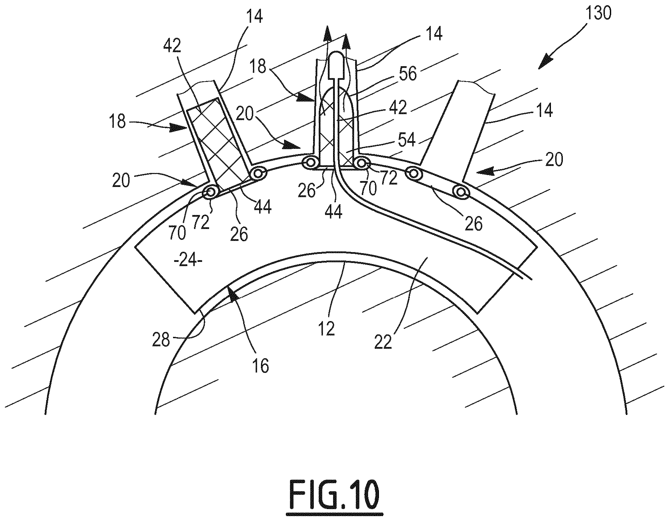

Un deuxième nécessaire 130 selon l'invention est illustré par la Figure 10. A la différence du premier nécessaire 10, le premier implant 16 du deuxième nécessaire 130 délimite une pluralité de fenêtres 26. Il comporte en outre, pour chaque fenêtre 26, un deuxième implant 18 destiné à être implanté dans la fenêtre 26, et un anneau élastique 20, rapporté sur le premier implant 16 autour de la fenêtre 26.

Le nécessaire 130 est par exemple destiné à être implanté dans la crosse aortique. Les différentes fenêtres 26 sont placées en regard des trois branches 14 présentes dans la crosse aortique.

Comme décrit précédemment, le premier implant 16 est déployé dans le tronçon principal 12 plaçant chaque fenêtre 26 en regard d'une branche 14. Puis, chaque deuxième implant 18 est implanté à travers une fenêtre 26 pour faire saillie dans une branche 14 comme décrit précédemment.

Compte tenu de la torsion importante du premier implant 16 dans le tronçon principal 12 et des orientations variables des branches 14, la présence d'un anneau élastique 20 autour de chaque fenêtre 26 garantit que l'étanchéité est réalisée autour de chaque deuxième implant 18, quelle que soit l'orientation relative du deuxième implant 18 par rapport au premier implant 16 et la conformation initiale de la fenêtre 26.

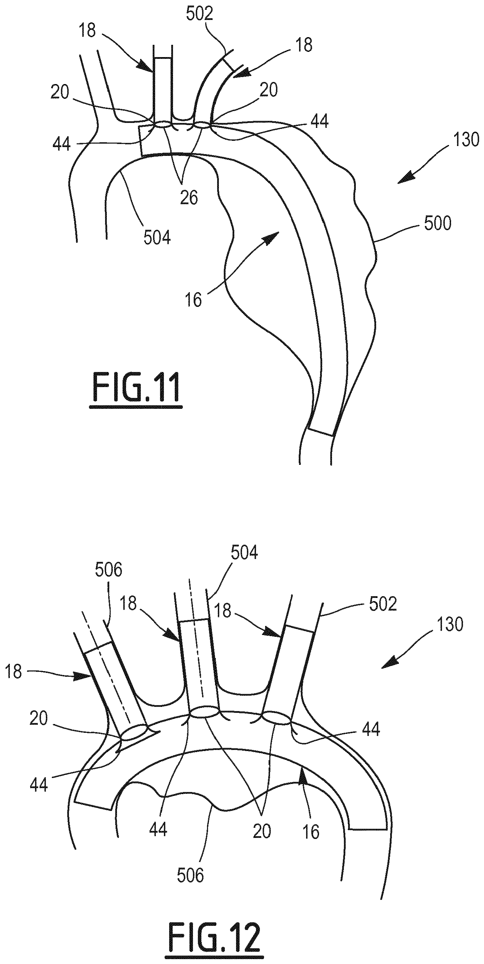

Une application du deuxième nécessaire 130 selon l'invention est illustrée par la Figure 11. Un tel nécessaire 130 est destiné à être implanté dans un anévrisme 500 de l'aorte thoracique descendante nécessitant de couvrir l'artère sous-clavière gauche 502 et l'artère carotide primitive gauche 504 pour disposer d'un collet d'ancrage suffisant. Dans cette configuration, l'ostia du vaisseau est le plus souvent contigu de la fenêtre 26.

Dans ce cas, le premier implant 16 comporte deux fenêtres 26 placées respectivement en regard de l'artère sous-clavière gauche et de l'artère carotide primitive. Les deuxièmes implants 18 sont introduits à partir des vaisseaux branchés à travers respectivement l'artère sous-clavière gauche et l'artère carotide primitive gauche jusqu'à chaque fenêtre 26.

Puis, les organes radiaux 44 des implants 18 respectifs sont déployés au sein de chaque anneau élastique 20 présents autour de la fenêtre 26. Le nécessaire 130 ainsi implanté bloque de manière très efficace la migration éventuelle du premier implant 16 qui est parfaitement immobilisé en partie dans la crosse aortique 504 et en partie dans l'anévrisme 500.

De plus, même si les artères 502, 504 présentent des angles variés avec l'axe local de la crosse aortique, l'étanchéité entre chaque deuxième implant 18 et le premier implant 16 au niveau des fenêtres 26 est garantie par la présence des anneaux élastiques 20. Le risque de migration du premier implant 16 est en outre limité au minimum par la force substantielle de retenue engendrée par les anneaux 20.

Une autre application du deuxième nécessaire 130 selon l'invention est illustrée par la Figure 12. Dans cette application, le premier implant 16 est implanté à travers un anévrisme 506 de la crosse aortique 504 dans lequel les ostia des troncs supra-aortiques 502, 504, 508 sont distants des fenêtres 26 prévues dans le premier implant 16.

Les deuxièmes implants 18 sont introduits à travers les troncs 502, 504, 508 et exercent une traction sur le premier implant 16 par l'intermédiaire des anneaux élastiques 20 coopérant avec les organes de retenue 44 de chaque deuxième implant 18.

Ainsi, les deuxièmes implants 18 font remonter le premier implant 16 dans la crosse aortique 504 pour épouser la plus grande courbure de celle-ci et réduire le risque de migration et de fuite au niveau des collets. En effet, les axes des collets sont alignés avec l'implant 18.

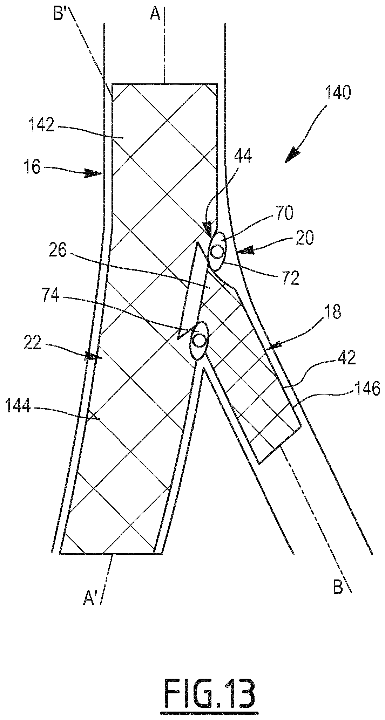

Un troisième nécessaire 140 selon l'invention est illustré par la Figure 13. Ce nécessaire 140 comporte un premier implant 16 présentant un tronc principal 142 et une première jambe 144. L'implant 16 délimite une fenêtre 26 à la base du tronc, située à l'opposé de la première jambe 144. Le deuxième implant 18 est destiné à être implanté dans la fenêtre 26 pour former une deuxième jambe 146.

Le nécessaire 140 forme ainsi une prothèse bifurquée qui présente généralement une forme de Y.

Dans cet exemple, l'angle formé entre l'axe local A-A' du premier implant 16 au niveau de la fenêtre 26 et l'axe B-B' du deuxième implant 18 est inférieur à 90° et est notamment compris entre 30° et 45°.

L'organe radial 44 dans sa position déployée est par ailleurs situé dans un plan incliné par rapport à l'axe local B-B' du deuxième implant 18. Grâce à l'anneau élastique 20 présent sur le premier implant autour de la fenêtre 26, l'étanchéité est donc assurée de manière très efficace autour du deuxième implant 18.

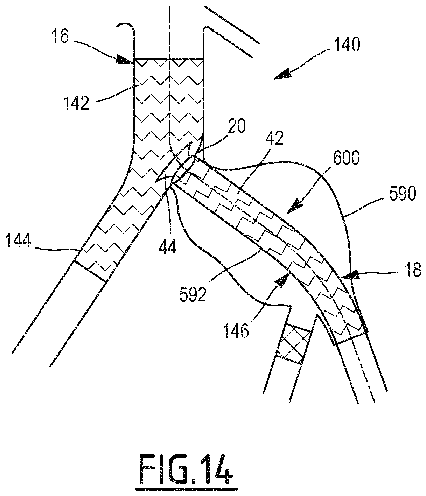

Une application du troisième nécessaire 140 selon l'invention est représentée sur la Figure 14. Dans cette application, le troisième nécessaire 140 est destiné par exemple à traiter un anévrisme 590 de l'artère illiaque primitive 592 chez un patient ne présentant pas d'anévrisme aortique associé et sans collet proximal permettant la mise en place d'une endoprothèse tubulaire dans l'artère illiaque 592.

Dans ce cas, le tronc principal 142 présente avantageusement un diamètre compris entre 15 mm et 25 mm et une hauteur comprise entre 2 cm et 3 cm pour ne pas couvrir l'origine de l'artère mésentérique supérieure.

La branche 146 présente un diamètre compris entre 8 mm et 14 mm et une longueur comprise entre 2 cm et 3 cm.

Le quatrième nécessaire selon l'invention 140 permet donc de produire des branches 146 avec des angulations préétablies, ce qui est très avantageux en pratique clinique.

Dans cette configuration, l'endoprothèse proximale comporte une partie proximale s'adaptant au diamètre de l'aorte du patient et une partie distale s'adaptant au diamètre de son artère iliaque proximale, la partie proximale et la partie distale étant d'un seul tenant. La longueur des parties proximales (aortique) et/ou distale (iliaque) peut être adaptée pour éviter de couvrir l'artère mésentérique inférieure et/ou l'artère iliaque interne.

Dans une variante, la surface extérieure du deuxième corps tubulaire 42 du deuxième implant 18 est munie d'un revêtement adhérent, par exemple en silicone au niveau de sa zone de contact avec l'anneau 20.

Le revêtement adhérent favorise la fixation du deuxième implant 18 dans l'anneau 20 au contact de l'enveloppe 72.

Dans une variante, l'enveloppe 72 de l'anneau élastique 20 est recouverte d'un revêtement étanche, avantageusement biodégradable. Ce revêtement est par exemple formé de nanofibres de PLA qui bouchent les orifices du tissu formant l'enveloppe 72.

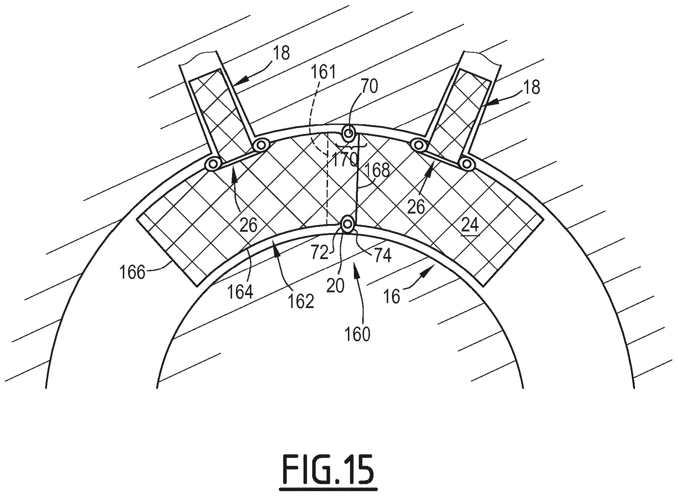

Un quatrième nécessaire 160 selon l'invention est illustré par la figure 15. A la différence du premier nécessaire 10, le premier implant 16 définit une fenêtre axiale 161 dans laquelle est inséré un troisième implant 162. La fenêtre axiale 161 s'étend à une extrémité axiale du premier implant 16 et est formée par une ouverture axiale débouchant dans le passage central 24.

Le premier implant 16 comporte un anneau de retenue additionnel 20 s'étendant autour de la fenêtre axiale 161. L'anneau de retenue additionnel 20 est déformable élastiquement dans la fenêtre 161 pour permettre une augmentation réversible d'au moins 20%, avantageusement d'au moins 30% du contour extérieur du passage d'introduction du troisième implant 161 défini par l'anneau de retenue additionnel 20.

L'anneau de retenue additionnel 20 est avantageusement du type décrit plus haut. Il comporte un ressort 70 circonférentiel et une enveloppe 72 délimitant un logement 74 de réception du ressort 70.

Le troisième implant 162 est destiné à être inséré à travers la fenêtre 161. Il présente un troisième corps tubulaire 164 s'étendant entre un premier bord axial 166 et un deuxième bord axial 168. Un tronçon axial 170 d'extrémité du troisième implant 162 est inséré à travers le passage central 24 du premier implant 16 à travers la fenêtre 161.

Comme décrit précédemment, l'anneau de retenue 20 se déforme autour de la surface extérieure du troisième corps tubulaire 164 pour réaliser une étanchéité autour de cette surface. Ainsi, le contour intérieur de l'anneau 20 présente une forme conjuguée à celle du contour extérieur du tronçon axial 170 du troisième implant 162 disposé dans le premier implant 16.

Avantageusement, le premier implant 16 et le troisième implant 164 délimitent chacun une fenêtre latérale 26 recevant un deuxième implant 18.

La présence d'un troisième implant 162 mobile axialement dans un premier implant 16, et retenu de manière étanche par un anneau de retenue 20 selon l'invention permet donc d'ajuster la position axiale relative des deuxièmes implants 18 l'un par rapport à l'autre, pour s'adapter à des conformations anatomiques différentes.

Ainsi, il n'est pas nécessaire de prévoir directement dans le premier implant 16 plusieurs fenêtres d'insertion 26 d'un deuxième implant 18 espacées axialement les unes des autres par une distance prédéterminée qui doit être prévue dès la conception de l'implant 16.

Il est au contraire possible de standardiser la position des fenêtres 26 sur chaque implant 16, 162, puis, d'implanter tout d'abord le premier implant 16, et ensuite d'insérer axialement le troisième implant 162 dans le premier implant 16 afin de placer la fenêtre 26 du troisième implant 162 à la distance choisie pour être située en regard du conduit anatomique spécifique de l'utilisateur.

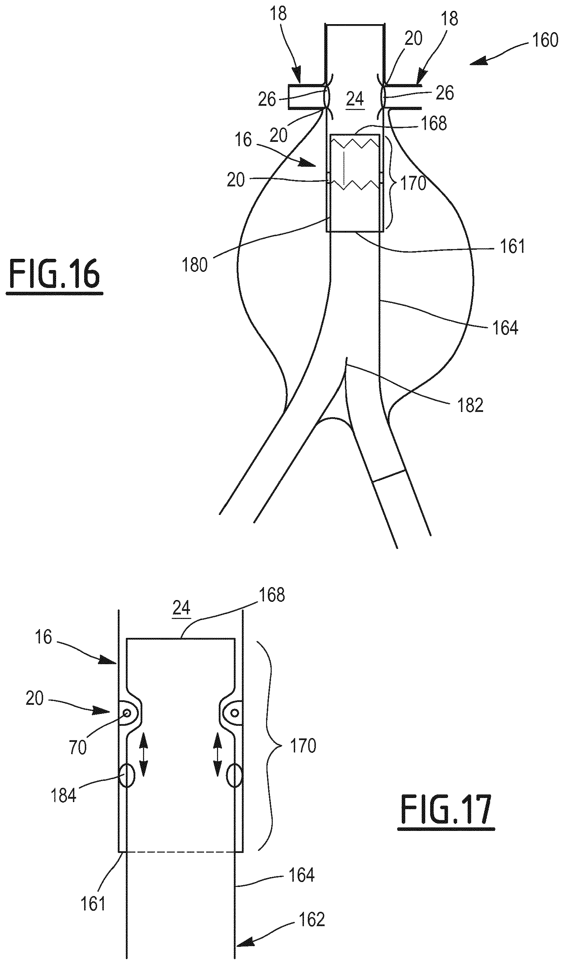

Un exemple d'application du quatrième nécessaire 160 selon l'invention est illustré par les figures 16 et 17.

Dans cet exemple, le premier implant 16 est disposé dans une partie linéaire de l'aorte sous rénale et dans l'aorte abdominale. Les deuxièmes implants 18 sont disposés dans les artères rénales à travers des fenêtres 26 munies d'anneaux élastiques 20 tels que décrits plus haut.

Le troisième implant 162 présente un tronc commun 180 inséré partiellement dans la fenêtre axiale 161 du premier implant 16 et une bifurcation 182 comprenant des jambes insérées dans les artères illiaques.

Un anneau élastique 20 additionnel est disposé dans le premier implant 16 autour de la fenêtre axiale 161. L'anneau 20 se déforme autour de la surface extérieure du troisième corps tubulaire 164 pour réaliser une étanchéité autour de cette surface. Ainsi, le contour intérieur de l'anneau 20 présente une forme conjuguée à celle du contour extérieur du tronçon axial 170 du troisième implant 162 disposé dans le premier implant 16.

Comme illustré sur la figure 17, l'anneau additionnel 20 déforme radialement le tronçon axial 170 vers l'axe du tronçon axial assurant une retenue axiale du troisième implant 162 par rapport au premier implant 16.

Ce raccordement, du fait de ses propriétés élastiques, s'adapte aux mouvements imposés par le flux systoliques. Le raccordement joue donc le rôle d'un amortisseur qui permet au tronçon aval du troisième implant 162, qui comporte la bifurcation, d'être l'objet de micromouvements imposés par les forces de trainée (« drag forces ») sans répercuter ces forces sur les zones qui comportent les branches 18 et les fenêtres 26 dans les artères rénales et d'éviter les risques de disjonctions.

Dans une variante, représentée en pointillés sur la figure 17, un coussinet 184 est disposé autour du tronçon axial 170 du troisième implant 162. Le coussinet 184 se place entre la fenêtre axiale 161 et l'anneau additionnel 20 lorsque le tronçon axial 170 du troisième implant 162 est introduit dans le premier implant 16.

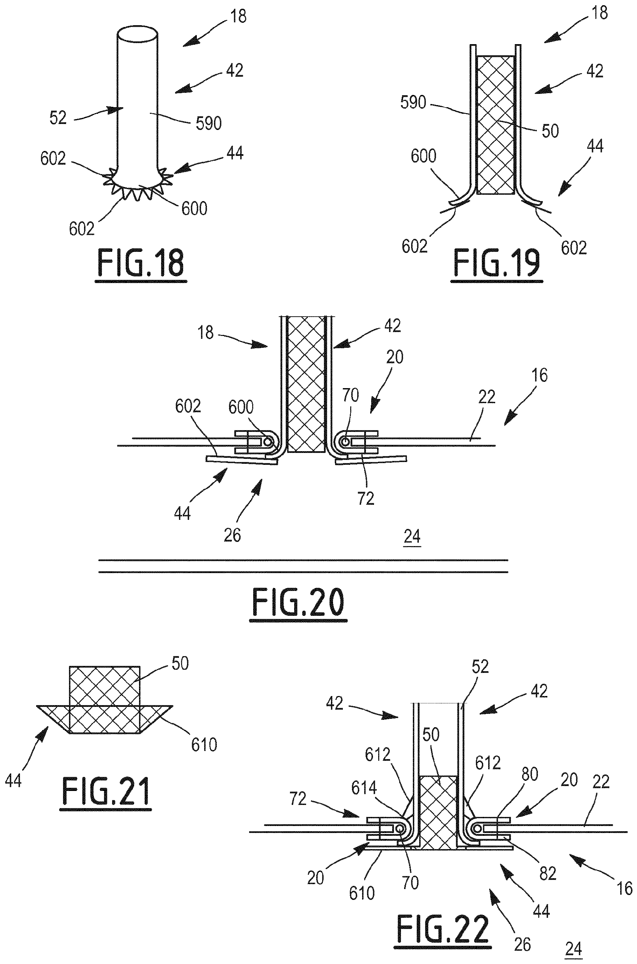

Des exemples de deuxièmes implants 18 destinés à être introduits dans une fenêtre 26 d'un premier implant 16 vont maintenant être décrits sur les figures 18 à 22.

Dans un premier exemple d'implant 18, représenté sur les figures 18 à 20, le corps tubulaire 42 de l'implant 18 comporte une armature tubulaire ajourée 50 et une couche extérieure 52 fixée autour de l'armature 50. La couche extérieure 52 présente une partie 590 fixée sur l'armature 50 et un évasement 600 qui fait saillie radialement et axialement à l'écart de l'armature 50.

L'organe radial de retenue 44 est fixé sur l'évasement 600. Il comporte dans cet exemple une pluralité de doigts 602 de retenue faisant saillie radialement.

Le deuxième implant 18 dans sa configuration rétractée est par exemple introduit dans la fenêtre 26 d'un premier implant 16 (voir Figure 20), à travers une branche latérale du vaisseau dans lequel est disposé le premier implant 16.

Une fois introduit dans le passage central 24, l'organe de retenue radial 44 est tout d'abord déployé, puis calé contre la surface intérieure du premier corps tubulaire 22 en regard de l'anneau de retenue 20.

Ensuite, le deuxième corps tubulaire 42 est déployé et s'applique contre l'anneau de retenue 20. Celui-ci se déforme alors élastiquement pour épouser la forme et l'orientation angulaire du deuxième implant 18.

Les figures 21 et 22 illustrent une variante de deuxième implant 18 pour un nécessaire selon l'invention.

Dans cette variante, l'organe de retenue radiale 44 est solidaire de l'armature 50. Il est en particulier venu de matière avec l'armature 50.

Comme illustré par la figure 21, l'organe de retenue radiale 44 est par exemple formé par une jupe tronconique faisant saillie radialement à partir de l'armature tubulaire 50. La jupe 610 est repliée axialement en regard de l'armature tubulaire 50. L'évasement 600 est fixé sur la jupe 610.

De plus, l'implant décrit sur la figure 21 peut être mis en place dans un lanceur de largage dans la configuration décrite sur cette figure 21 et être largué par principe de point-fixe retrait. Ceci présente le désavantage de doubler les épaisseurs de stent et de membrane. Pour pallier ce problème, l'implant 18 peut être disposé dans le lanceur non pas avec la jupe 610 repliée sur l'armature 50, mais avec la jupe 610 étendue en utilisant un lanceur spécifique. Ce lanceur comporte une butée escamotable sur laquelle est poussée l'armature 50 pour permettre l'évasement de la jupe 610. Ensuite, la butée est escamotée et le reste de l'armature 18 est largué par principe de point-fixe retrait.

Lors de la mise en place de l'implant 18 dans la fenêtre 26, la jupe 610 se cale sous l'anneau 20 en s'appliquant sur la surface intérieure du corps tubulaire 22.

Avantageusement, le deuxième implant 18 comprend en outre des pattes 612 de calage de l'organe de retenue 44, visibles sur la figure 22. Les pattes 612 font saillie à partir de la surface extérieure du deuxième corps 42 vers l'organe de retenue 44, en regard de celui-ci.

Les pattes 612 de calage et l'organe de retenue 44 délimitent ainsi un espace intermédiaire 612 de calage de l'anneau 20.

Lorsque le deuxième implant 18 est placé dans la fenêtre 26, l'anneau 20 est enserré entre les pattes 612 disposées sur la collerette extérieure 80 et l'organe de retenue 44 appliqué sur la collerette intérieure 82.

La retenue du deuxième implant 18 par rapport au premier implant 16 est donc très robuste.

Dans l'invention qui vient d'être décrite, l'anneau de retenue 20 est déformable élastiquement dans la fenêtre 26 pour permettre une augmentation réversible d'au moins 20% du contour extérieur du passage d'introduction 60.

Ceci constitue une différence par rapport aux dispositifs de l'état de la technique, dans lesquel l'anneau de retenue est faiblement déformable, par exemple avec une déformabilité inférieure à 10%, et dans lequel un deuxième implant déployé par un ballon gonflable doit être utilisé pour compenser la rétraction éventuelle du deuxième implant, après dégonflage du ballon.

La présence d'une déformation élastique réversible d'au moins 20% du contour extérieur du passage d'introduction permet d'accommoder des implants 18 qui sont autoexpansibles, et de garantir l'étanchéité autour du deuxième implant 18, quelle que soit l'orientation ou la configuration du deuxième implant 18. On produit ainsi un système d'affrontement de deux structures élastiques qui s'opposent mécaniquement permettant non seulement de générer une très importante force d'affrontement, mais également une adaptation géométrique de l'affrontement. Ceci n'est pas le cas, si on met une structure déformée plastiquement dans une collerette à faible pouvoir de déformation comme dans l'état de la technique.

L'étanchéité est en outre améliorée par l'organe de retenue 44 du deuxième implant 18, notamment lorsque cet organe 14 est une collerette, cette collerette pouvant coopérer avec l'anneau de retenue 20. Lorsque l'anneau de retenue 20 est muni sur sa surface extérieure d'un revêtement d'étanchéité, et éventuellement d'un revêtement adhérent, notamment en silicone, l'étanchéité est encore améliorée.

Dans une forme de réalisation préférée de l'invention, l'anneau de retenue comporte une enveloppe 72 formée par une première collerette 80 fixée sur une surface extérieure du premier corps 22, une deuxième collerette 82 fixée sur une surface intérieure du premier corps 22 et une paroi circonférentielle 84 raccordant la première collerette à la deuxième collerette 82.

De préférence, les collerettes 80, 82 et la paroi circonférentielle 84 sont venues de matière.

L'enveloppe 72 rapportée sur le premier corps tubulaire 22 du premier implant 16 simplifie considérablement la fabrication du nécessaire de traitement selon l'invention, par rapport aux nécessaires décrits dans l'état de la technique. En particulier, la configuration particulière de la collerette, avec un profil torique ouvert vers l'extérieur formant une gouttière radiale permet de loger de manière sûre l'organe élastique 70 présent dans l'anneau de retenue 20, tout en garantissant la déformabilité de l'anneau 20 dans la gamme revendiquée.

L'enveloppe 72 étant préformée dans cette configuration et rapportée dans la fenêtre 26, les contraintes appliquées sur la paroi du premier implant 16 sont minimes, ce qui garantit la solidité de l'implant 16 autour de la fenêtre 26.

La mise en place de l'anneau de retenue 20 est par ailleurs particulièrement simple à réaliser industriellement.

Ceci constitue un avantage majeur par rapport aux solutions dans lesquelles une éversion de la paroi de l'implant est réalisée pour former un anneau de retenue. Ces solutions présentent en effet le désavantage d'être difficiles à réaliser industriellement, d'induire des contraintes majeures sur le corps de l'implant, et de s'opposer à la libre déformation de l'anneau de retenue.

Dans une variante avantageuse, un limiteur d'extension, formé par exemple par un fil rigide non déformable élastiquement, est introduit dans l'anneau de retenue 20 pour limiter l'augmentation réversible du contour extérieur du passage d'introduction 60.

Le limiteur est par exemple introduit dans l'organe élastique 70, par exemple au centre du ressort. La déformation élastique maximale de l'anneau de retenue 20 est alors limitée, ce qui garantit toujours une utilisation de l'organe élastique 70 dans sa gamme de déformabilité réversible.

## Revendications

1. - Nécessaire de traitement (10 ; 130 ; 140, 160) comportant :
- un premier implant (16), le premier implant (16) comportant un premier corps tubulaire (22) définissant une fenêtre (26) ;
- un deuxième implant (18) comportant un deuxième corps tubulaire (42) destiné à être disposé dans la fenêtre (26) du premier corps tubulaire (22) pour faire saillie par rapport au premier implant (16), le deuxième implant (18) comportant un organe (44) de retenue du deuxième implant (18) par rapport au premier implant (16) ;
- un anneau (20) de retenue du deuxième implant (18) rapporté sur le premier corps tubulaire (22) dans la fenêtre (26) et délimitant un passage d'introduction (60) du deuxième implant (18),
l'anneau de retenue (20) étant déformable élastiquement dans la fenêtre (26) pour permettre une augmentation réversible d'au moins 20 %, avantageusement d'au moins 30 % du contour extérieur du passage d'introduction (60) et l'anneau de retenue (20) comportant une enveloppe (72) rapportée sur le premier corps (22) autour de la fenêtre (26), l'enveloppe (72) définissant un logement (74) s'étendant au moins partiellement autour de la fenêtre (26), l'anneau de retenue (20) comportant un organe élastique (70), notamment un ressort disposé dans le logement (74), l'organe élastique (70) présentant avantageusement une forme annulaire, **caractérisé en ce que** l'enveloppe (72) comporte une collerette extérieure (80), une collerette intérieure (82) et une paroi tubulaire (84) centrale raccordant les bords intérieurs (86) des collerettes (80, 82), et la paroi tubulaire (28) du corps tubulaire (22) est reçue partiellement dans le logement (74) défini entre les collerettes (80, 82).

2. - Nécessaire (10 ; 130 ; 140 ; 160) selon la revendication 1, **caractérisé en ce que** le deuxième implant (18) est mobile par rapport au premier implant (16) entre une position de repos située à l'écart de la fenêtre (26) et une position active implantée dans la fenêtre (26), l'anneau de retenue (20) étant déformé élastiquement lorsque le deuxième corps (42) est disposé dans la fenêtre (26), le contour intérieur du passage d'introduction (60) étant de forme conjuguée au contour extérieur du deuxième corps (42) situé au contact de l'anneau de retenue (20).

3. - Nécessaire (10 ; 130 ; 140 ; 160) selon la revendication 1, **caractérisé en ce que** l'organe élastique (70) est un ressort hélicoïdal, avantageusement fermé sur lui-même, le ressort hélicoïdal (70) délimitant une ouverture centrale (76).

4. - Nécessaire (10 ; 130 ; 140 ; 160) selon l'une quelconque des revendications 1 ou 3, **caractérisé en ce que** l'enveloppe (72) comporte une première collerette (80) fixée sur une surface extérieure du premier corps (22), une deuxième collerette (82) fixée sur une surface intérieure du premier corps (22) et une paroi circonférentielle (84) raccordant la première collerette (80) à la deuxième collerette (82), le logement (74) étant délimité entre la première collerette (80), la deuxième collerette (82) et la paroi circonférentielle (84), le ressort (70) s'étendant autour de la paroi circonférentielle (84).

5. - Nécessaire (10 ; 130 ; 140 ; 160) selon la revendication 4, **caractérisé en ce que** la première collerette (80), la deuxième collerette (82) et la paroi circonférentielle (84) sont venues de matière, et sont avantageusement formées en tissu tressé ou tricoté, tel qu'un tissu de polyéthylène téréphtalate ou de polytétrafluoroéthylène.

6. - Nécessaire (10 ; 130 ; 140 ; 160) selon l'une quelconque des revendications précédentes, **caractérisé en ce que** l'organe de retenue (44) du deuxième implant (18) est formé par une collerette (58) radiale déployable.

7. - Nécessaire (10 ; 130 ; 140 ; 160) selon l'une quelconque des revendications précédentes, **caractérisé en ce que** le deuxième implant (18) est autoexpansible entre une configuration rétractée et une configuration déployée constituant sa configuration de repos.

8. - Nécessaire (10 ; 130 ; 140 ; 160) selon l'une quelconque des revendications précédentes, **caractérisé en ce que** le premier corps (22) est une endoprothèse tubulaire, le deuxième corps (42) étant une endoprothèse tubulaire.

9. - Nécessaire (10 ; 130 ; 140) selon l'une quelconque des revendications précédentes, **caractérisé en ce que** le premier corps tubulaire (22) délimite un passage central (24) s'étendant suivant un axe longitudinal entre une première ouverture axiale et une deuxième ouverture axiale, la fenêtre (26) étant une fenêtre latérale débouchant transversalement par rapport à l'axe longitudinal entre les ouvertures axiales.

10. - Nécessaire (10 ; 130 ; 140) selon l'une quelconque des revendications précédentes, **caractérisé en ce que** l'anneau de retenue (20) comporte un limiteur d'extension.

11. - Dispositif de traitement comportant un premier implant (16) comprenant un premier corps tubulaire (22) définissant une fenêtre (26), et un anneau (20) de retenue d'un deuxième implant (18), rapporté dans la fenêtre (26) et délimitant un passage (60) d'introduction du deuxième implant (18), l'anneau de retenue (20) étant déformable élastiquement dans la fenêtre pour permettre une augmentation réversible d'au moins 20 %, avantageusement d'au moins 30 % du contour extérieur du passage d'introduction (60), et l'anneau de retenue (20) comportant une enveloppe (72) rapportée sur le premier corps (22) autour de la fenêtre (26), l'enveloppe (72) définissant un logement (74) s'étendant au moins partiellement autour de la fenêtre (26), l'anneau de retenue (20) comportant un organe élastique (70), notamment un ressort disposé dans le logement (74), l'organe élastique (70) présentant avantageusement une forme annulaire, **caractérisé en ce que** l'enveloppe (72) comporte une collerette extérieure (80), une collerette intérieure (82) et une paroi tubulaire (84) centrale raccordant les bords intérieurs (86) des collerettes (80, 82), et la paroi tubulaire (28) du corps tubulaire (22) étant reçue partiellement dans le logement (74) défini entre les collerettes (80, 82).

12. Procédé de fabrication d'un dispositif selon la revendications 11, **caractérisé en ce qu'**il comporte les étapes suivantes :
- fourniture d'un premier implant (16) comportant un premier corps tubulaire (22) définissant une fenêtre (26) ;
- fixation d'un anneau de retenue (20) d'un deuxième implant (18) dans la fenêtre (26), l'anneau de retenue (20) délimitant un passage d'introduction (60) d'un deuxième implant (18), **caractérisé en ce que** l'anneau de retenue (20) est déformable élastiquement dans la fenêtre (26) pour permettre une augmentation réversible d'au moins 20 %, avantageusement d'au moins 30 % du contour extérieur du passage d'introduction (60), et **en ce qu'**il comporte une étape de fabrication de l'anneau de retenue (20), l'étape de fabrication comportant la fourniture d'une enveloppe (72) définissant un logement (74), et la mise en place d'un l'organe élastique (70), notamment d'un ressort dans le logement (74) de l'enveloppe (72), l'étape de fixation de l'anneau de retenue (20) comportant la fixation de l'enveloppe (72) sur le premier corps (22).

13. - Procédé selon la revendication 12, **caractérisé en ce que** l'étape de fixation de l'enveloppe (72) comporte la fixation d'une première collerette (80) sur une surface extérieure du premier corps (22), la fixation d'une deuxième collerette (82) sur une surface intérieure du premier corps (22) et la fourniture d'une paroi circonférentielle (84) raccordant la première collerette (80) à la deuxième collerette (82), le logement (74) étant délimité entre la première collerette (80), la deuxième collerette (82) et la paroi circonférentielle (84), l'organe élastique (70) étant disposé autour de la paroi circonférentielle (84).

14. - Procédé selon l'une quelconque des revendications 12 ou 13, **caractérisé en ce qu'**il comporte une étape de fabrication de l'enveloppe (72), comportant :
- la formation d'un tube (109), avantageusement en tissu ;
- la création d'un étranglement (110) dans le tube (109) ;
- le figeage de l'étranglement (110) dans le tube (109) pour former le logement (74).

## Patentansprüche

1. Behandlungskit (10; 130; 140, 160), das enthält:
- ein erstes Implantat (16), wobei das erste Implantat (16) einen ersten röhrenförmigen Körper (22) enthält, der ein Fenster (26) definiert;
ein zweites Implantat (18), das einen zweiten röhrenförmigen Körper (42) enthält, der dazu bestimmt ist, im Fenster (26) des ersten röhrenförmigen Körpers (22) untergebracht zu werden, um gegenüber dem ersten Implantat (16) herauszuragen, das zweite Implantat (18) enthält ein Halteelement (44) des zweiten Implantats (18), bezogen auf das erste Implantat (16);
- einen Haltering (20) des zweiten Implantats (18), angesetzt auf dem ersten röhrenförmigen Körper (22) im Fenster (26), der eine Einführungspassage (60) des zweiten Implantats (18) begrenzt,
- der Haltering (20) ist dabei elastisch im Fenster (26) verformbar, um eine umkehrbare Vergrößerung um mindestens 20 %, besser noch mindestens 30 % des Außenumfangs der Einführungspassage (60) zu ermöglichen und der Haltering (20) enthält eine Hülle (72), angesetzt auf dem ersten Körper (22), um das Fenster (26) herum, die Hülle (72) definiert dabei eine Aussparung (74), die wenigstens teilweise um das Fenster (26) herum liegt, der Haltering (20) enthält ein elastisches Element (70), insbesondere eine Feder, angeordnet in der Aussparung (74), das elastische Element (70) weist vorteilhafterweise eine Ringform auf, **dadurch gekennzeichnet, dass** die Hülle (72) einen Außenbund (80), einen Innenbund (82) und eine zentrale röhrenförmige Wand (84) enthält, die die Innenränder (86) der Bünde (80, 82) verbindet und die röhrenförmige Wand (28) des röhrenförmigen Körpers (22) teilweise in der zwischen den beiden Bünden (80, 82) definierten Aussparung (74) untergebracht ist.

2. - Kit (10; 130; 140; 160) nach Anspruch 1, **dadurch gekennzeichnet, dass** das zweite Implantat (18) gegenüber dem ersten Implantat (16) beweglich ist zwischen einer Ruheposition, die sich im Abstand vom Fenster (26) befindet und einer aktiven Position, untergebracht im Fenster (26), der Haltering (20) wird dabei elastisch verformt, wenn der zweite Körper (42) im Fenster (26) angeordnet ist, der Innenumfang der Einführungspassage (60) entspricht dem des Außenumfangs des zweiten Körpers (42), der sich in Kontakt zum Haltering (20) befindet.

3. Kit (10; 130; 140; 160) nach Anspruch 1, **dadurch gekennzeichnet, dass** das elastische Element (70) eine Spiralfeder ist, vorteilhafterweise in sich geschlossen, wobei die Spiralfeder (70) eine zentrale Öffnung umgrenzt (76).

4. Kit (10; 130; 140; 160) nach einem beliebigen der Ansprüche 1 oder 3, **dadurch gekennzeichnet, dass** die Hülle (72) einen ersten Bund (80) enthält, der auf einer Außenfläche des ersten Körpers (22) befestigt ist, ein zweiter Bund (82), befestigt auf einer Innenfläche des ersten Körpers (22) und eine umlaufende Wand (84), die den ersten Bund (80) mit dem zweiten Bund (82) verbindet, die Aussparung (74) wird dabei zwischen dem ersten Bund (80), dem zweiten Bund (82) und der umlaufenden Wand (84) begrenzt, die Feder (70) verläuft entlang der umlaufenden Wand (84).

5. Kit (10; 130; 140; 160) nach Anspruch 4, **dadurch gekennzeichnet, dass** der erste Bund (80), der zweite Bund (82) und die umlaufende Wand (84) aus demselben Material bestehen,vorteilhafterweise aus geflochtenem oder gewirkten Material, wie ein Polyethylenterephtalat- oder Polytetrafluorethylen - Gewebe.

6. Kit (10; 130; 140; 160) nach irgendeinem der vorangehenden Ansprüche, **dadurch gekennzeichnet, dass** das Halteelement (44) des zweiten Implantats (18) von einem ausfaltbaren radialen Bund (58) gebildet wird.

7. Kit (10; 130; 140; 160) nach irgendeinem der vorangehenden Ansprüche, **dadurch gekennzeichnet, dass** das zweite Implantat (18) selbstexpandierend ist, zwischen einer eingefahrenen Konfiguration und einer ausgefahrenen Konfiguration, die seine Ruhekonfiguration darstellt.

8. Kit (10; 130; 140; 160) nach irgendeinem der vorangehenden Ansprüche, **dadurch gekennzeichnet, dass** der erste Körper (22) eine röhrenförmige Endoprothese ist, der zweite Körper (42) ist eine röhrenförmige Endoprothese.

9. Kit (10; 130; 140) nach irgendeinem der vorangehenden Ansprüche, **dadurch gekennzeichnet, dass** der erste röhrenförmige Körper (22) eine zentrale Passage (24) umgrenzt, die entlang einer Längsachse zwischen einer ersten axialen Öffnung und einer zweiten axialen Öffnung verläuft, bei dem Fenster (26) handelt es sich um ein Seitenfenster, das quer zur Längsachse zwischen den axialen Öffnungen mündet.

10. Kit (10; 130; 140) nach einem beliebigen der vorangehenden Ansprüche, **dadurch gekennzeichnet, dass** der Haltering (20) eine Ausdehnungsbegrenzung enthält.

11. Behandlungsvorrichtung mit einem ersten Implantat (16), umfassend einen ersten röhrenförmigen Körper (22), der ein Fenster (26) definiert, und einem Haltering (20) eines zweiten Implantats (18), eingesetzt im Fenster (26), der die Einführungspassage (60) eines zweiten Implantats (18) begrenzt, der Haltering (20) ist dabei elastisch im Fenster verformbar, um eine umkehrbare Vergrößerung um mindestens 20 %, noch besser mindestens 30 % des Außenumfangs der Einführungspassage (60) zu erlauben, und der Haltering (20) enthält dabei eine Hülle (72), angesetzt am ersten Körper (22) um das Fenster (26) herum, die Hülle (72) definiert dabei eine Aussparung (74), die zumindest zum Teil um das Fenster (26) herum verläuft, der Haltering (20) enthält dabei ein elastisches Element (70), insbesondere eine Feder, die in der Aussparung (74) untergebracht ist, das elastische Element (70) weist vorteilhafterweise ein Ringform auf, **dadurch gekennzeichnet, dass** die Hülle (72) einen Außenbund (80), einen Innenbund (82) und eine zentrale, röhrenförmige Wand (84) enthält, die die Innenränder (86) der Bünde (80, 82) verbindet, die röhrenförmige Wand (28) des röhrenförmigen Körpers (22) wird teilweise in der Aussparung (74) aufgenommen, die zwischen den Bünden (80, 82) definiert ist.

12. Verfahren zur Herstellung einer Vorrichtung nach Anspruch 11, **dadurch gekennzeichnet, dass** es die folgenden Schritte enthält:
- Bereitstellung eines ersten Implantats (16) mit einem ersten röhrenförmigen Körper (22), der ein Fenster (26) definiert;
- Befestigung des Halterings (20) eines zweiten Implantats (18) im Fenster (26), der Haltering (20) begrenzt eine Einführungspassage (60) für ein zweites Implantat (18), **dadurch gekennzeichnet, dass** der Haltering (20) elastisch im Fenster (26) verformbar ist, um eine umkehrbare Vergrößerung um mindestens 20 %, noch besser mindestens 30 % des Außenumfangs der Einführungspassage (60) zu erlauben, und dadurch, dass er den Schritt der Herstellung eines Halterings (20) enthält, der Schritt der Herstellung umfasst die Bereitstellung einer Hülle (72), die eine Aussparung (74) definiert und das Einsetzen eines elastischen Elementes (70), insbesondere einer Feder in der Aussparung (74) der Hülle (72), der Schritt der Befestigung des Halterings (20) enthält dabei die Befestigung der Hülle (72) auf dem ersten Körper (22).

13. Verfahren nach Anspruch 12, **dadurch gekennzeichnet, dass** der Schritt der Befestigung der Hülle (72) die Befestigung eines ersten Bundes (80) auf einer Außenfläche des ersten Körpers (22) umfasst, die Befestigung eines zweiten Bundes (82) auf einer Innenfläche des ersten Körpers (22) und die Bereitstellung einer umlaufenden Wand (84), die den ersten Bund (80) mit dem zweite Bund (82) verbindet, die Aussparung (74) ist dabei zwischen dem ersten Bund (80), dem zweiten Bund (82) und der umlaufenden Wand (84) begrenzt, das elastische Element (70) ist um die umlaufende Wand (84) herum angeordnet.

14. Verfahren nach einem beliebigen der Ansprüche 12 oder 13, **dadurch gekennzeichnet, dass** es einen Schritt der Herstellung der Hülle (72) enthält, die umfasst:
- die Bildung eines Schlauchs (109), vorteilhafterweise aus Gewebe;
- die Bildung eines Engpasses (110) im Schlauch (109);
- das Erstarren des Engpasses (110) im Schlauch (109) um die Aussparung zu bilden.

## Claims

1. Treatment kit (10; 130; 140, 160) comprising
- a first implant (16), the first implant (16) comprising a first tubular body (22) defining a window (26);
- a second implant (18) comprising a second tubular body (42) intended to be disposed in the window (26) of the first tubular body (22) so as to protrude relative to the first implant (16), the second implant (18) comprising a member (44) for retaining the second implant (18) with respect to the first implant (16);
- a ring (20) for retaining the second implant (18) assembled on the first tubular body (22) in the window (26) and delimiting a passage (60) for introducing the second implant (18),
the retaining ring (20) being elastically deformable in the window (26) to enable a reversible increase of at least 20%, advantageously of at least 30% of the outer contour of the introduction passage (60), and the retaining ring (20) comprising
an envelope (72) assembled on the first body (22) around the window (26), the envelope (72) defining a housing (74) extending at least partially around the window (26), the retaining ring (20) comprising an elastic member (70), in particular a spring disposed in the housing (74), the elastic member (70) advantageously having an annular shape,
**characterised in that** the envelope (72) comprises an outer collar (80), an inner collar (82) and a central tubular wall (84) connecting the inner edges (86) of the collars (80, 82), and the tubular wall (28) of the tubular body (22) is partially received in the housing (74) defined between the collars (80, 82).

2. Kit (10; 130; 140; 160) according to claim 1, **characterised in that** the second implant (18) is movable with respect to the first implant (16) between a rest position located away from the window (26) and an active position implanted in the window (26), the retaining ring (20) being elastically deformed when the second body (42) is disposed in the window (26), the inner contour of the introduction passage (60) having a shape conjugated with the outer contour of the second body (42) situated in contact with the retaining ring (20).

3. Kit (10; 130; 140; 160) according to claim 1, **characterised in that** the elastic member (70) is a helical spring, advantageously closed on itself, the helical spring (70) delimiting a central opening (76).

4. Kit (10; 130; 140; 160) according to any one of claims 1 or 3, **characterised in that** the envelope (72) comprises a first collar (80) fastened on an outer surface of the first body (22), a second collar (82) fastened on an inner surface of the first body (22) and a circumferential wall (84) connecting the first collar (80) to the second collar (82), the housing (74) being delimited between the first collar (80), the second collar (82) and the circumferential wall (84), the spring (70) extending around the circumferential wall (84).

5. Kit (10; 130; 140; 160) according to claim 4, **characterised in that** the first collar (80), the second collar (82) and the circumferential wall (84) are formed as one piece, and are advantageously formed of braided or knitted fabric, such as a polyethylene terephthalate or polytetrafluoroethylene fabric.

6. Kit (10; 130; 140; 160) according to any one of the preceding claims, **characterised in that** the retaining member (44) of the second implant (18) is formed by a deployable radial collar (58).

7. Kit (10; 130; 140; 160) according to any one of the preceding claims, **characterised in that** the second implant (18) is auto-expandable between a retracted configuration and a deployed configuration constituting its rest configuration.

8. Kit (10; 130; 140; 160) according to any one of the preceding claims, **characterised in that** the first body (22) is a tubular endoprosthesis, the second body (42) being a tubular endoprosthesis.

9. Kit (10; 130; 140) according to any one of the preceding claims, **characterised in that** the first tubular body (22) delimits a central passage (24) extending along a longitudinal axis between a first axial opening and a second axial opening, the window (26) being a lateral window emerging transversally relative to the longitudinal axis between the axial openings.

10. Kit (10; 130; 140) according to any one of the preceding claims, **characterised in that** the retaining ring (20) comprises an extension limiter.

11. Treatment device comprising a first implant (16) comprising a first tubular body (22) defining a window (26), and a ring (20) for retaining a second implant (18), assembled in the window (26) and delimiting a passage (60) for introducing the second implant (18), the retaining ring (20) being elastically deformable in the window to enable a reversible increase of at least 20%, advantageously of at least 30% of the outer contour of the introduction passage (60), and the retaining ring (20) comprising an envelope (72) assembled on the first body (22) around the window (26), the envelope (72) defining a housing (74) extending at least partially around the window (26), the retaining ring (20) comprising an elastic member (70), in particular a spring disposed in the housing (74), the elastic member (70) advantageously having an annular shape, **characterised in that** the envelope (72) comprises an outer collar (80), an inner collar (82) and a central tubular wall (84) connecting the inner edges (86) of the collars (80, 82), and the tubular wall (28) of the tubular body (22) being partially received in the housing (74) defined between the collars (80, 82).

12. Method for manufacturing a device according to claim 11, **characterised in that** it comprises the following steps:
- providing a first implant (16) comprising a first tubular body (22) defining a window (26);
- fastening a ring (20) for retaining a second implant (18) in the window (26), the retaining ring (20) delimiting a passage (60) for introducing a second implant (18), **characterised in that** the retaining ring (20) is elastically deformable in the window (26) to enable a reversible increase of at least 20%, advantageously of at least 30% of the outer contour of the introduction passage (60), and **in that** it comprises a step for fabricating the retaining ring (20), the fabrication step comprising the provision of an envelope (72) defining a housing (74), and the placement of an elastic member (70), in particular a spring in the housing (74) of the envelope (72), the step of fastening the retaining ring (20) comprising the fastening of the envelope (72) on the first body (22).

13. Method according to claim 12, **characterised in that** the step of fastening the envelope (72) comprises the fastening of a first collar (80) on an outer surface of the first body (22), the fastening of a second collar (82) on an inner surface of the first body (22) and the provision of a circumferential wall (84) connecting the first collar (80) to the second collar (82), the housing (74) being delimited between the first collar (80), the second collar (82) and the circumferential wall (84), the elastic member (70) being disposed around the circumferential wall (84).

14. Method according to any one of claims 12 or 13, **characterised in that** it comprises a step of fabricating the envelope (72), comprising:
- the formation of a tube (109), advantageously made of fabric;
- the creation of a throttle (110) in the tube (109);
- the freezing of the throttle (110) in the tube (109) to form the housing (74).
